# EUROPEAN PATENT SPECIFICATION

(11) **EP 2 160 233 B1**
(45) Date of publication and mention of the grant of the patent: **17.10.2012**
(21) Application number: 08774196.3
(22) Date of filing: 20.06.2008
(51) Int. Cl.: B01D 53/04, C07C 7/148, C08F 10/00

(54) **PROCESS FOR THE POLYMERISATION OF OLEFINS**
VERFAHREN ZUR POLYMERISIERUNG VON OLEFINEN
PROCÉDÉ DE POLYMÉRISATION D'OLÉFINES

(30) Priority: 22.06.2007 EP 07110842
(43) Date of publication of application: 10.03.2010
(73) Proprietor: TOTAL RESEARCH & TECHNOLOGY FELUY, 7181 Seneffe (BE)
(72) Inventor: HORTMANN, Kai, B-1700 Dilbeek (BE); VANDEWIELE, David, B-7110 Strépy-Bracquegnies (BE); GAUTHIER, William J., Houston, Texas 77062 (US)
(74) Representative: Leyder, Francis
(86) International application number: PCT/EP2008/057906
(87) International publication number: WO 2009/000782

(56) References cited:
- EP-A1- 0 683 147
- WO-A-03/048087
- US-A- 5 470 456

## Description

### FIELD OF THE INVENTION

The present invention relates to a process for the polymerisation of olefins. More particularly, the present invention relates to a process for the polymerisation of propylene.

### BACKGROUND OF THE INVENTION

As is well known, olefins, in particular ethylene and propylene, are used to produce numerous types of intermediate and end products, which are predominantly polymeric materials. Various types of catalysts can be used for the polymerisation process. However, due to improved product qualities, metallocene catalysts are becoming increasingly prevalent in industry.

Unfortunately, metallocene catalysts are also very sensitive to any quality variations that occur in the hydrocarbon feedstocks. As a result, catalyst activities become irregular, productivity decreases, and the full potential of the metallocene cannot be exploited. The fluctuating catalyst activities mean that polymer properties, such as the melt flow index, fluctuate as well. Hence, one is forced to continuously adjust the reaction conditions and reactant feed rates, which may not always lead to positive results. Such adjustments generally increase the production of off-specification resin and add to the economic damage of high catalyst costs.

Several methods are known in the art, which attempt to stabilise the polymerisation process. These include, for instance, cryogenic distillation, liquid adsorption and membrane separation of the olefin feedstock prior to polymerisation. However these processes have the disadvantages of high capital cost and high operating expenses. Furthermore, these processes do not stabilise polymerisation processes adequately enough.

US 4,830,735, BE 9000332, EP 0308569, EP 0643028 and EP 0648720 disclose contacting a hydrocarbon feedstock with an absorbent comprising nickel and nickel oxide to improve Ziegler-Natta catalyst activity. None of these documents disclose metallocene-type polymerisations.

US 2003/0105376 discloses passing olefins over two heterogeneous sorbents comprising of various metals and metal oxides prior to polymerisation with various catalysts. The first sorbent removes acetylenic impurities, the second carbon monoxide. However, there does not appear to be any stabilisation of polymerisation conditions.

EP 0683147 discloses contacting olefins with alkali metal carried on a support material prior to polymerisation with a metallocene. An example of polymerisation of the thus purified propylene using a Ziegler-Natta catalyst is provided showing that the catalytic activity increases when using the purified propylene. However, the sorbent includes metallic alkali metal and hence the olefin must be dried before the sorbent can be contacted with the olefin stream. Furthermore, no mention is made of stabilising the polymerisation conditions.

JP 05070373A2 discloses the purification of propylene using metallic nickel for the purpose of polymerising the propylene. After treatment, the hydrocarbon stream contains less than 0.1 parts per million (ppm) each of carbon monoxide and carbonyl sulphide. However, as it can be seen in the examples the amounts of carbonyl sulphide and carbon monoxide still fluctuate after purification, reducing the catalyst's activity and providing polymers of unstable melt indices. Furthermore, polymerisation in connection with metallocenes is not disclosed.

Accordingly, it can be seen that there is a need for a stabilised metallocene-type polymerisation process with high productivity.

### SUMMARY OF THE INVENTION

The present invention is directed to a process for the metallocene-type polymerisation of olefin-containing hydrocarbon feedstock that has been passed over a sorbent material comprising nickel deposited on a support material wherein said nickel is present as both nickel oxide and metallic nickel.

### DETAILED DESCRIPTION OF THE INVENTION

The present invention relates to a process for the metallocene-type polymerisation of olefins. While the subsequent discussion will describe the invention in terms of polymerising propylene, it should be understood that the present invention may be applicable to the polymerisation of other olefins e.g. ethylene, propylene, butenes, pentenes, hexenes, octenes or any combinations thereof. The method is preferably applied to the polymerisation of propylene due to the advantages of propylene's physical properties.

The process of this invention comprises the steps of:
a. passing an olefin-containing hydrocarbon feedstock over a sorbent material comprising nickel deposited on a support material wherein said nickel is present as both nickel oxide and metallic nickel.
b. converting at least part of the olefins contained in said hydrocarbon feedstock into a polymer over one or more metallocene catalysts.
c. recovering the polymer product.

### The sorbent material

Without wishing to be bound by any theory, it is believed that impurities in the olefin feedstock are substantially removed when passing it over a sorbent material comprising metallic nickel and nickel oxide. The resulting hydrocarbon feedstock is thus more suitable for stable metallocene-type polymerisations. Silica, silico-aluminas, alumina, kieselguhr, zeolites and other similar materials, whether amorphous or crystalline, can be utilised as the support. The total weight of nickel and nickel oxide may represent from 10 wt. % up to 80 wt.% of the sorbent material. Accordingly, the sorbent material may include 20 to 90 wt.% of support material. Preferably, the weight ratio of metallic nickel to nickel oxide is of 0.4 to 2.0, with the provision that metallic nickel should neither represent less than 6 wt.%, nor more than 50 wt.% of the sorbent material, and the sorbent material comprises from 40 to 70 wt.% of metallic nickel and nickel oxide and from 30 to 60 wt.% of support material. When carrying out the process of the invention with a sorbent material outside this definition, the polymerisation results obtained may no longer be satisfactory, although polymerisation conditions may be somewhat stabilised. Whilst not wishing to be bound by any theory, the Applicant believes that larger crystallites are formed if the nickel to nickel oxide ratio is higher, thus leading to a lower efficiency; similarly, an excessive total nickel content tends to lower the specific surface and consequently the efficiency, while a too low total nickel content would lead to an insufficient capacity for sorbing impurities.

The nickel can be deposited on the support by any of the several methods well known to those skilled in the art. For example, nickel can be deposited on the support by dissolving nickel nitrate in water, mixing the solution with the support and precipitating the nickel, for example in the form of nickel carbonate, and subsequently washing, drying and calcining the precipitate. The nickel deposited in this manner is then partially reduced by means of hydrogen to form metallic nickel, the remainder being in the form of nickel oxide.

In general, the size of the nickel crystallites after reduction is from 1 to 2 nm. The size of the nickel crystallites depends on the extent of the reduction carried out. In fact, if the degree of reduction is increased, the size of the crystallites is increased but the sorbent material obtained does not have the desired properties. On the other hand, if the degree of reduction is too low, the crystallites still have good dimensions but the quantity of nickel available in this case is too small to ensure successful treatment of the feedstock.

The specific surface area of the sorbent material obtained after reduction is generally between 100 and 200 m²/g.

The particle size of the sorbent material depends especially on the pressure drop allowed in the reactor; it has been noted, however, that it is advantageous to use the sorbent material in finely divided form. Preferably, the particle diameter of this material when spherical does not exceed about 3.5 mm and is most preferably from 1 to 2.5 mm. When cylindrical particles are used, they preferably have a diameter of from 1 to 2 mm and a length from 3 to 8 mm. Trilobes of similar size may also be used.

The sorbent material is usually prepared ex situ and stored either under a convenient saturated liquid hydrocarbon, like cyclohexane or dodecane, or under a non-oxidizing atmosphere like N₂. It can also be protected by deposition of a carbon dioxide layer on the surface, said layer protecting the sorbent material from air, thus allowing easy handling. However, the carbon dioxide layer must be removed before use of the material, e.g. by nitrogen stripping at about 200°C.

It has been found that propylene adsorbs onto the sorbent material when contacted with the feedstocks, and that the propylene adsorption reaction, occurring during start-up, is exothermic. Under certain conditions, and particularly when the sorbent material is stored under a non-oxidizing atmosphere, the temperature rise may be very important. More particularly the temperature at the surface of the material may be much higher than that measured with a thermocouple, and the sorbent material may thus be damaged. In addition, the high temperatures trigger undesired side-reactions, more particularly propylene dimerisation and trimerisation. The dimers are hexenes, which may or may not copolymerise with propylene. If this propylene stream is then used for polymerisations, these by-products can break the regularity of the linear chain of isotactic polypropylene or otherwise cause process difficulties. As a result, the copolymer has a lower crystallinity than polypropylene, and thus a lower melting point; its mechanical resistance is also lower. More seriously, during polymerisation these dimers also act as retarders by blocking active sites on the catalyst, thereby significantly reducing productivity and increasing associated catalyst costs.

The Applicants have found that an excessive increase in the temperature of the sorbent material can be avoided by conditioning it before use. Conditioning can be carried out by passing an inert gas flow containing a minor amount of at least one light olefin, preferably propylene in a concentration of from 0.1 to 5 vol %, over said material. The inert gas is usually nitrogen, which should contain the least possible amount of oxygen. It is preferable to begin the conditioning procedure by passing essentially pure inert gas over the material. The conditioning step is preferably carried out at about atmospheric pressure, at or below ambient temperature. It is continued until the propylene concentration at the outlet equals that introduced. It is also possible to monitor the passage of an exotherm, shown by thermocouples introduced within the sorbent material.

It is known that, when the sorbent material is prepared ex situ and protected by a monolayer of carbon dioxide (believed to be sorbed onto the nickel surface) the sorbent material must be pre-treated prior to its conditioning by passing an initial inert gas (containing the least possible amount of oxygen) over it at a temperature of from about 150°C to about 350°C, preferably at about 250°C and preferably at about atmospheric pressure. This is then preferably followed by passing a mixture of inert gas and hydrogen containing an increasing concentration of hydrogen over it (to remove any oxygen possibly sorbed despite all precautions), before purging it free of hydrogen with an inert gas flow at about 250°C.

The process of the present invention is capable of improving productivities and activities of metallocene catalysts by reducing the concentration of impurities present in the olefin-containing hydrocarbon feed. The original concentration of impurities may be as high as 1000 ppm or higher depending on the process used to produce the original feedstock. However, in such cases, it is usually more economical to carry out in advance known purification processes such as distillation or the use of molecular sieves to reduce the content of impurities prior to the process of the present invention.

Whilst not wishing to be bound by any theory, the Applicant believes that the metallic nickel and nickel oxide combination reacts with impurities present in the olefin-containing feedstock. The amounts of impurities fluctuate from olefin source to olefin source, thereby causing catalyst activities to fluctuate as well. One of these impurities may possibly be carbon monoxide, a potent metallocene catalyst poison. By substantially removing impurities such as carbon monoxide from the hydrocarbon feedstock, the catalyst activity can remain constant. Hence, polymerisation conditions no longer need to be continuously adjusted and the polymer product melt flow indices are stabilised. It is also thought that the nickel-based sorbent may also act as a hydrogenation catalyst, removing substantial amounts of hydrogen from the propylene source and forming propane. As a result, melt flow indices are stabilised.

Once spent, the sorbent can be partially reactivated by treatment with inert gas at elevated temperatures and optionally with addition of hydrogen.

In polyolefin production, the hydrocarbon feedstock generally comprises more than 75 wt.% of olefins, more particularly from 85 to 99.99 wt.%. In one embodiment of the present invention, the olefin-containing hydrocarbon feedstock is passed over the sorbent material at a temperature of from -10°C to 80°C, preferably of from 0°C to 40°C, more preferably from 0°C to 30°C, more preferably from 0°C to 25°C, even more preferably 0°C to 20°C and at a liquid hourly space velocity (LHSV) of from 0.1 to 60 I/I.h, namely of from 5, 10, 20, 25, 30, 35 or 40 up to 45, 50, 55 or 60 I/I.h, preferably at a LHSV of from 20 to 60 I/I.h, more preferably of from 20 to 40 I/I.h, and most preferably at about 30 I/I.h. It is surprising that still even at temperatures as low as 0°C to 30°C, more surprisingly as low as 0°C to 25°C and 0°C to 20°C and at high LHSVs of from 20 to 40 I/I.h or even to 60 I/I.h, but in particular at around 30 I/I.h, the nickel sorbent still provides an important stabilisation of catalyst activities.

Optionally, additional sorbents may be used in combination with the nickel sorbent. The feedstock may be passed over one or more additional sorbents prior to passing it over the nickel sorbent. These act as guard beds and as a result, the nickel sorbent's overall lifetime is increased. One or more additional sorbents can also be used after the nickel sorbent bed. The additional sorbents can be any sorbent known to a person skilled in the art. Examples of possible additional sorbents are metal oxides such as copper oxide, zinc oxide, zirconium oxide or manganese oxide, aluminas (including promoted aluminas), palladium, platinum, and molecular sieves such as 3A, 4A, 5A or 13X, as well as copper/copper oxide sorbents. Preferably, molecular sieve 13X is used, because of its larger pore size.

### Polymerisation

The polymerisation step of the inventive process can be any known metallocene-type polymerisation. Any metallocene catalyst known in the art can be used. It is represented by the general formula:
I. (CpRₙ)ₘ M Xq
   wherein Cp is a substituted or non-substituted cyclopentadienyl ring, M is a transition metal from Group IVB of the Periodic Table or vanadium, each R is the same or different and is a hydrocarbyl or hydrocarboxyl radical having from 1 to 20 carbon atoms, each X is the same or different and may be a hydrocarbyl or hydrocarboxy radical having 1-20 carbon atoms or a halogen, and m=1-3, n=0-5, q=0-3 and the sum m+q is equal to the oxidation state of the metal.
II. (C₅R'ₖ)_{g}R"ₛ(C₅R'ₖ)MQ_{3-g}
II R"ₛ(C₅R'ₖ)₂ MQ' wherein (C₅R'ₖ) is a cyclopentadienyl or substituted cyclopentadienyl, each R' is the same or different and is hydrogen or a hydrocarbyl radical such as alkyl, alkenyl, aryl, alkylaryl, or arylalkyl radical containing from 1 to 20 carbon atoms or two carbon atoms are joined together to form a C₄-C₆ ring, R" is a C₁-C₄ alkylene radical, a dialkyl germanium or silicon or siloxane, or a alkyl phosphine or amine radical bridging two (C₅R'ₖ) rings, Q is a hydrocarbyl radical such as aryl, alkyl, alkenyl, alkylaryl, or aryl alkyl radical having from 1-20 carbon atoms, hydrocarboxy radical having 1-20 carbon atoms or halogen and can be the same or different from each other, Q' is an alkylidene radical having from 1 to about 20 carbon atoms, s is 0 or 1, g is 0, 1 or 2.

Examples of metallocenes that can be used among others are ethylene bis(tetrahydroindenyl) zirconium dichloride, ethylene bis(indenyl) zirconium dichloride or bis(n-butylcyclopentadienyl) zirconium dichloride, dimethylsilylbis(2-methyl-4-phenylindenyl)zirconium dichloride, dimethylsilylbis(2-methylindenyl)zirconium dichloride, dimethylsilylbis(2-methyl-4,5-benzoindenyl)zirconium dichloride, isopropylidene(cyclopentadienyl)(fluorenyl)zirconium dichloride, isopropylidene(2-methyl-4-tert-butyl-cyclopentadienyl)(fluorenyl)zirconium dichloride, isopropylidene-(2-methyl-4-tert-butyl-cyclopentadienyl)(3,6-ditertbutyl-fluorenyl)zirconium dichloride. Mixtures of different metallocene catalysts can be used if multi-modal polymers are desired. These can be present together in one reactor or separately in a number of reactors, which are connected in series or in parallel.

The cocatalyst, which activates the metallocene catalyst component, can be any cocatalyst known for this purpose such as an aluminium-containing cocatalyst, a boron-containing cocatalyst or a fluorinated catalyst. The aluminium-containing cocatalyst may comprise an alumoxane, an alkyl aluminium, a Lewis acid and/or a fluorinated catalytic support.

The alumoxanes that may be used in the process of the present invention are well known by the person skilled in the art and preferably comprise oligomeric linear and/or cyclic alkyl alumoxanes represented by the formula: 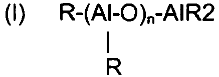 for oligomeric, linear alumoxanes and 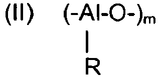 for oligomeric, cyclic alumoxane,
wherein n is 1-40, preferably 10-20, m is 3-40, preferably 3-20 and R is a C₁-C₈ alkyl group and preferably methyl.

Generally, in the preparation of alumoxanes, for example, methylalumoxane (MAO), a mixture of linear and cyclic compounds is obtained.

When alumoxane is not used as the cocatalyst, one or more aluminiumalkyl represented by the formula AlRₓ are used wherein each R is the same or different and is selected from halogens or from alkoxy or alkyl groups having from 1 to 12 carbon atoms and x is from 1 to 3. Especially suitable are trialkylaluminiums, the most preferred being triisobutylaluminium (TIBAL) and triethylaluminum (TEAL). Suitable boron-containing cocatalysts may comprise a triphenylcarbenium boronate such as tetrakis-pentafluorophenyl-borato-triphenylcarbenium as described in EP 0427696, or those of the general formula [L'-H] + [B Ar₁ Ar₂ X₃ X₄]- as described in EP 0277004 (page 6, line 30 to page 7, line 7).

The metallocene catalyst system may be employed in a solution polymerisation process, which is homogeneous, or a slurry process, which is heterogeneous. In a solution process, typical solvents include hydrocarbons with 4 to 7 carbon atoms such as heptane, toluene or cyclohexane. In a slurry, bulk or gas-phase process it is necessary to immobilise the catalyst system on an inert support. The metallocene may be supported according to any method known in the art. In the event it is supported, the support can be any organic or inorganic solid. Particularly porous solid supports such as talc, inorganic oxides, and resinous support material such as polyolefin are preferred. More preferably, the support material is an inorganic oxide in its finely divided form. Examples of useful supported catalysts are described in US 6,855,783.

Suitable inorganic oxide materials, which are desirably employed in accordance with this invention, include Group IIa, IIIa, IVa or IVb metal oxides such as silica, alumina and mixtures thereof. Other inorganic oxides that may be employed either alone or in combination with the silica, or alumina are magnesia, titania, zirconia. Other suitable support materials, however, can be employed, for example, finely divided functionalised polyolefins such as finely divided polyethylene. Preferably, the support is a silica having a surface area between 200 and 900 m²/g and a pore volume between 0.5 and 4 ml/g.

The amount and order of addition of alumoxane, if used, and metallocenes employed in the preparation of the solid support catalyst can vary. Preferably the aluminium to transition metal mole ratio is in the range between 1:1 and 1000:1, preferably in the range 5:1 and 500:1. In accordance with a preferred embodiment of the present invention alumoxane dissolved in a suitable inert hydrocarbon solvent is added to the support material slurried in the same or other suitable hydrocarbon liquid and thereafter a mixture of the metallocene catalyst component is added to the slurry.

Diluents, which can be used for the slurry polymerisation process, include mineral oils and various hydrocarbons which are liquid at reaction temperature and which do not react with the individual ingredients. Illustrative examples of the useful solvents include the alkanes such as pentane, iso-pentane, n-hexane, isohexane, heptane, octane and nonane; cycloalkanes such as cyclopentane and cyclohexane; and aromatics such as benzene, toluene, xylene, ethylbenzene and diethylbenzene.

The process of polymerisation using a metallocene catalyst is well known in the art. In accordance with the invention, purified olefin from the first step and optionally an alpha-olefinic comonomer purified in the same manner, are supplied to the reactor containing the metallocene catalyst. Comonomer is usually added to control the density and reduce the melting temperature of the resulting polymer. Typical comonomers include ethylene, propylene, 1-hexene, 1-butene, 1-octene or 4-methylpentene. Preferably, 1-hexene is used as the comonomer for ethylene polymerisation and ethylene is used as the comonomer for propylene polymerisation. As is known by the person skilled in the art, the average molecular weight is controlled using hydrogen.

Where the reaction is performed in a slurry using, for example, isobutane, a reaction temperature in the range of 70°C to 110°C may be used. The reaction can also be carried out in a bulk process i.e. where the diluent and monomer are the same. Where the reaction is performed in solution, by selection of a suitable solvent a reaction temperature in the range 150°C to 300°C may be used. The reaction may also be performed in the gas phase using a suitably supported catalyst.

In order to exploit the full potential of metallocene catalysts propylene and ethylene polymerisations, it is essential to have stable polymerisation conditions. Only then will metallocene productivity be high and constant. It has been unexpectedly found that by passing the hydrocarbon feedstock over a sorbent material as hereinbefore described, a higher metallocene catalyst productivity can be achieved. Furthermore, the catalyst productivities, and hence catalyst flow rates, remain more constant, thus allowing for better control over the polymer products. Secondly, there is enhanced melt flow control.

The following examples are given, though by way of illustration only, to show preferred processes of the present invention. These examples should not, however, be construed as limiting the scope of the claimed invention as there are many variations which may be made thereon, as those skilled in the art will recognise.

### EXAMPLES OF THE INVENTION

### Examples E1 and E2 and Comparative Example CE3

Polymerisation of propylene was performed using a standard metallocene isotactic polypropylene (miPP) catalyst as described in US 6,855,783. A 3-litre reactor was charged with 1.5 litres of propylene and 0.4 NL of hydrogen. The catalyst (0.3 ml of 20wt% catalyst slurried in oil) was brought into contact with 69 mg of TEAL for 5 minutes and added to the reactor with the addition of 0.5 litres of propylene. The polymerisation temperature was maintained at 70°C for 1 hour, after which, the contents of the reactor were flared and the polymer isolated. Propylene used in this study was either directly injected into the reactor or first passed through a sorbent bed containing nickel and nickel oxide. The results of the polymerisation with and without passage through the nickel sorbent bed are shown in Table 1. The productivity is provided in relative terms. The results indicate that with the same catalyst injected into the reactor in the same amounts, the productivity increases 33-to 34-fold when the propylene is passed through the nickel - nickel oxide sorbent bed prior to polymerisation.

**Table 1**

| **Example** | **Sorbent** | **Yield / (g)** | **Relative Productivity** |
|---|---|---|---|
| E1 | Nickel / Nickel oxide | 480 | 34 |
| E2 | Nickel / Nickel oxide | 468 | 33 |
| CE3 | None | 14 | 1 |

### Examples E4-E8

Polymerisations were conducted with propylene sampled from different sources. The same catalyst (miPP) as in Examples E1, E2 and CE3 was used. The purpose was to verify that the use of nickel-nickel oxide sorbents provide benefits beyond a single supply of propylene. Five different sources (from 1 to 5) of propylene were evaluated. Results were compared with passages through 13X molecular sieves alone and without any sorbent. Table 2 shows that the sorbent of the invention provides an excellent improvement in productivity, regardless of the source of the propylene and is typically higher than 13X alone. The productivity is provided in relative terms.

**Table 2**

| **Ex.** | **Propylene source** | **Relative miPP productivity: No sorbent** | **Relative miPP productivity: Nickel-nickel oxide sorbent** | **Relative miPP productivity: 13X sorbent** |
|---|---|---|---|---|
| E4 | 1 | 12.7 | 13.9 | 10.7 |
| E5 | 2 | 6.3 | 12.8 | 11.3 |
| E6 | 3 | 1 | 14.8 | 6.1 |
| E7 | 4 | 3.5 | 14.4 | 8.7 |
| E8 | 5 | 12.8 | 15.0 | 13.0 |

### Examples E9 and Comparative Example CE10

In Example E9, a polymerisation of propylene was conducted using propylene that had been passed over two 13X sorbent beds, followed by a nickel-nickel oxide sorbent bed. The same catalyst (miPP) as in Examples E1, E2 and CE3 was used to polymerise the propylene. The polymerisation process was stable and catalyst flow rates were constant, thus providing polypropylene having smaller variations in melt flow indices (MFI). Results are provided in Table 3 below. MFI was measured according to ASTM D1238 under a load of 2.16 kg and at a temperature of 230°C. Delta MFI represents the difference between the highest and the lowest MFI measured during polymerisation of propylene for a target MFI of 25. Activities are provided in relative terms.

In Comparative Example CE10, the same polymerisation process was carried out using propylene that had not been passed over the sorbent bed of the invention. The same metallocene catalyst (miPP) as in Examples E1, E2 and CE3 was used in combination with a similar metallocene catalyst (miPP*) known to have higher activities than miPP under laboratory conditions. However, despite this, the overall activity of the catalyst was almost half that of Example E9. Furthermore, the polymerisation conditions were not stable. Problems were encountered including instability in the high pressure flash lines and increased fluff. Furthermore, bulk density decreased considerably, which resulted in lower throughput and higher energy, i.e. kilowatt, consumption.

Table 3 shows that the delta MFI is smaller for Example E9 according to the invention (i.e. when the nickel - nickel oxide sorbent bed is used) than for Comparative Example CE10. This indicates that the MFI of the polypropylene obtained from the polymerisation process according to the invention vary less and are therefore more stable. Proof of the more stable reaction conditions is also provided by the delta hydrogen factors (the difference between the highest and lowest hydrogen concentrations measured during the run in ppm), whereby the hydrogen concentration varied more in Comparative Example CE10 than in Example E9.

**Table 3**

| **Ex.** | **Catalyst** | **Relative miPP activity** | **Average bulk / density (g/cm³)** | **Delta hydrogen factor / (ppm)** | **Delta MFI/ (dg/min)** |
|---|---|---|---|---|---|
| E9 | miPP | 1.86 | 0.42 | 30 | 6 |
| CE10 | miPP+miPP* | 1 | 0.39 | 150 | 13.1 |

### Example E11 and Comparative Examples CE12 and CE13

A polymerisation process was carried out according to the invention to show the improvement in the MFI stability of the polypropylene thereby obtained. Example E11 was conducted with the same metallocene catalyst (miPP) as used in Examples E1, E2 and CE3 using propylene that had been passed over one 13X sorbent bed, followed by two nickel-nickel oxide sorbent beds. The polymerisation conditions were stable and activity was constant. No problems with fines were observed. Results are shown in Table 4. Delta MFI is the difference between the highest and the lowest MFI measured during polymerisation of propylene. Example E11 provides data of two polypropylenes that were targeted, one with an MFI of 15, the other an MFI of 25. Activities are provided in relative terms.

In Comparative Example CE12 propylene was polymerised in the presence of the same catalyst as in Examples E1, E2 and CE3 (miPP). In Comparative Example CE13 propylene was polymerised using catalyst miPP* as described for Comparative Example CE10. Propylene was not passed over any nickel/nickel-oxide sorbent beds prior to polymerisation and as a result, the average activities in Comparative Examples CE12 and CE13 were much lower than that of Example E11. Table 4 shows the delta MFI of two polypropylenes, which have a target MFI of 15 and 25 respectively, are smaller in Example E11 according to the invention than in Comparative Examples CE12 and CE13. This means that the polymerisation process according to the invention is much more stable and thus providing polypropylene having less MFI variations.

**Table 4**

| **Ex.** | **Catalyst** | **Relative miPP activity** | **Average bulk density / (g/cm³)** | **Delta MFI / (dg/min) of target resin MFI = 15 dg/min** | **Delta MFI / (dg/min) of target resin MFI = 25 dg/min** |
|---|---|---|---|---|---|
| E11 | miPP | 1.83 | 0.42 very stable | 5.1 | 3.9 |
| CE12 | miPP | 1.32 | 0.42 | 7.4 | 11.8 |
| CE13 | miPP* | 1 | 0.42 | 9.4 | 5.8 |

Furthermore, Table 5 demonstrates that the standard error and standard deviation are higher for the MFI of propylene polymerised according to the prior art.

**Table 5**

| | **Target MFI / (dg/min)** | **MFI standard error** | **MFI standard deviation** |
|---|---|---|---|
| E11 | 25 | 0.29 | 1.08 |
| CE13 | 25 | 0.44 | 1.70 |

## Claims

1. A process for the polymerisation of olefins comprising the steps of:
a. passing an olefin-containing hydrocarbon feedstock over a sorbent material comprising nickel deposited on a support material wherein said nickel is present as both nickel oxide and metallic nickel;
b. converting at least part of the olefins contained in said hydrocarbon feedstock into a polymer over one or more metallocene catalysts; and
c. recovering the polymer product.

2. The process according to claim 1, wherein the total weight of nickel oxide and metallic nickel represents from 10 to 80 wt.% of the sorbent material, and the sorbent material includes 20 to 90 wt.% of the support material.

3. The process according to claim 1 or 2, wherein the weight ratio of metallic nickel to nickel oxide is from 0.4 to 2.0, with the provision that metallic nickel should neither represent less than 6 wt.%, nor more than 50 wt.% of the sorbent material, and the sorbent material comprises from 40 to 70 wt.% of metallic nickel and nickel oxide and from 30 to 60 wt.% of support material.

4. The process according to any one of the preceding claims, wherein said sorbent material has a specific surface area from 100 to 200 m²/g.

5. The process according to any one of the preceding claims, wherein the feedstock comprises more than 75 wt.% of propylene, preferably from 85 to 99.99 wt.%.

6. The process according to claim 5 wherein the metallocene is one of dimethylsilylbis(2-methyl-4-phenylindenyl)zirconium dichloride, dimethylsilylbis(2-methylindenyl)zirconium dichloride, dimethylsilylbis(2-methyl-4,5-benzoindenyl)zirconium dichloride, isopropylidene-(cyclopentadienyl)(fluorenyl)zirconium dichloride, isopropylidene(2-methyl-4-tert-butyl-cyclopentadienyl)(fluorenyl)zirconium dichloride, isopropylidene(2-methyl-4-tert-butyl-cyclopentadienyl)(3,6-ditertbutyl-fluorenyl)zirconium dichloride or a mixture thereof.

7. The process according to any one of claims 1 to 4, wherein the feedstock comprises more than 80% of ethylene, preferably from 90 to 99.99 wt.%.

8. The process according to claim 7, wherein the metallocene is one of ethylene bis(tetrahydroindenyl) zirconium dichloride, ethylene bis(indenyl) zirconium dichloride, bis(n-butylcyclopentadienyl) zirconium dichloride or a mixture thereof.

9. The process according to any one of the preceding claims, wherein during step (a) the temperature is from -10 to 80°C and the LHSV is from 0.1 to 60 I/I.h.

10. The process according to any one of the preceding claims, wherein at any stage prior to step (b), the olefin-containing hydrocarbon feedstock is passed over one or more of the following:
- molecular sieves, preferably 13X molecular sieves
- metal oxides
- alumina

## Patentansprüche

1. Verfahren zum Polymerisieren von Olefinen, umfassend die folgenden Schritte:
a. Passieren eines olefinhaltigen Kohlenwasserstoffrohmaterials über ein Sorptionsmaterial, umfassend Nickel, das auf einem Trägermaterial abgelagert ist, wobei das Nickel sowohl als Nickeloxid als auch metallisches Nickel vorhanden ist;
b. Umsetzen zumindest eines Teils der im Kohlenwasserstoffrohmaterial enthaltenen Olefine in ein Polymer an einem oder mehreren Metallocenkatalysatoren; und
c. Gewinnen des Polymerprodukts.

2. Verfahren nach Anspruch 1, wobei das Gesamtgewicht des Nickeloxids und des metallischen Nickels 10 bis 80 Gew.-% des Sorptionsmaterials repräsentieren, und wobei das Sorptionsmaterial 20 bis 90 Gew.-% des Trägermaterials enthält.

3. Verfahren nach Anspruch 1 oder 2, wobei das Gewichtsverhältnis von metallischem Nickel zu Nickeloxid 0,4 bis 2,0 beträgt, unter der Maßgabe, dass das metallische Nickel weder weniger als 6 Gew.-% noch mehr als 50 Gew.-% des Sorptionsmaterials repräsentieren sollte, und das Sorptionsmaterial 40 bis 70 Gew.-% metallisches Nickel und Nickeloxid und 30 bis 60 Gew.-% Trägermaterial umfasst

4. Verfahren nach einem der vorstehenden Ansprüche, wobei das Sorptionsmaterial einen spezifischen Oberflächenbereich von 100 bis 200 m²/g aufweist.

5. Verfahren nach einem der vorstehenden Ansprüche, wobei das Rohmaterial mehr als 75 Gew.-% Propylen, vorzugsweise 85 bis 99,9 Gew.-%, umfasst.

6. Verfahren nach Anspruch 5, wobei das Metallocen eines von Dimethylsilylbis(2-methyl-4-phenylindenyl)zirkoniumdichlorid, Dimethylsilylbis(2-methylindenyl)zirkondiumdichlorid, Dimethylsilylbis(2-methyl-4,5-benzoindenyl)zirkoniumdichlorid, Isopropyliden-(cyclopentadienyl)(fluorenyl)zirkoniumdichlorid, Isopropyliden(2-methyl-4-tert-butyl-cyclopentadienyl)(fluorenyl)zirkoniumdichlorid, Isopropyliden(2-methyl-4-tert-butyl-cyclopentadienyl)(3,6-diterbutyl-fluorenyl)zirkoniumdichlorid oder ein Gemisch davon ist.

7. Verfahren nach einem der Ansprüche 1 bis 4, wobei das Rohmaterial mehr als 80 % Ethylen, vorzugsweise 90 bis 99,99 Gew.-%, umfasst.

8. Verfahren nach Anspruch 7, wobei das Metallocen eines von Ethylenbis(tetrahydroindenyl)zirkoniumdichlorid, Ethylenbis(indenyl)zirkoniumdichlorid, Bis(n-butylcyclopentadienyl)zirkoniurndichlorid oder ein Gemisch davon ist.

9. Verfahren nach einem der vorstehenden Ansprüche, wobei während Schritt (a) die Temperatur -10 bis 80 °C beträgt und sich die LHSV auf 0,1 1 bis 60 l/l.h. beläuft.

10. Verfahren nach einem der vorstehenden Ansprüche, wobei das olefinhalfige Kohlenwasserstoffrohmaterial in einer beliebigen Phase vor Schritt (b) über eines des Folgenden passiert wird:
- Molekularsiebe, vorzugsweise 13X-Molekularsiebe
- Metalloxide
- Aluminiumoxid.

## Revendications

1. Procédé de polymérisation d'oléfines comprenant les étapes suivantes :
a. le passage d'une matière première hydrocarbonée contenant des oléfines sur un matériau sorbant comprenant du nickel déposé sur un matériau support, ledit nickel étant présent à la fois sous la forme d'oxyde de nickel et de nickel métallique ;
b. la conversion d'au moins une partie des oléfines contenues dans ladite matière première hydrocarbonée en un polymère sur un ou plusieurs catalyseurs métallocènes; et
c. la récupération du produit polymère.

2. Procédé selon la revendication 1, dans lequel le poids total d'oxyde de nickel et de nickel métallique représente de 10 à 80% en poids du matériau sorbant, et le matériau sorbant comprend 20 à 90 % en poids du matériau support.

3. Procédé selon la revendication 1 ou 2, dans lequel le rapport en poids entre le nickel métallique et l'oxyde de nickel est de 0,4 à 2,0, à condition que le nickel métallique ne représente ni moins de 6 % en poids, ni plus de 50% en poids du matériau sorbant, et le matériau sorbant comprend de 40 à 70 % en poids de nickel métallique et d'oxyde de nickel, et de 30 à 60 % en poids de matériau support.

4. Procédé selon l'une quelconque des revendications précédentes, dans lequel ledit matériau sorbant a une aire de surface spécifique de 100 à 200 m²/g.

5. Procédé selon l'une quelconque des revendications précédentes, dans lequel la matière première comprend plus de 75 % en poids de propylène, de préférence de 85 à 99,99 % en poids.

6. Procédé selon la revendication 5, dans lequel le métallocène est le dichlorure de diméthylsilylbis(2-méthyl-4-phénylindényl)zirconium ou le dichlorure de diméthylsilylbis(2-méthylindényl)zirconium ou le dichlorure de diméthylsilylbis(2-méthyl-4,5-benzoindényl)zirconium ou le dichlorure d'isopropylidène-(cyclopentadiényl)(fluorényl)zirconium ou le dichlorure d'isopropylidène(2-méthyl-4-tert-butyl-cyclopentadiényl)(fluorényl)zirconium ou le dichlorure d'isopropylidène(2-méthyl-4-tert-butyl-cyclopentadiényl)(3,6-ditertbutyl-fluorényl)zirconium ou un de leurs mélanges.

7. Procédé selon l'une quelconque des revendications 1 à 4, dans lequel la matière première comprend plus de 80 % d'éthylène, de préférence de 90 à 99,99 % en poids.

8. Procédé selon la revendication 7, dans lequel le métallocène est le dichlorure d'éthylène bis(tétrahydroindényl)zirconium ou le dichlorure d'éthylène bis(indényl)zirconium ou le dichlorure de bis(n-butylcyclopentadiényl)zirconium ou un de leurs mélanges.

9. Procédé selon l'une quelconque des revendications précédentes, dans lequel, pendant l'étape (a), la température est de -10 à 80 °C et la LHSV est de 0,1 à 60 l/l·h.

10. Procédé selon l'une quelconque des revendications précédentes, dans lequel, à un moment quelconque avant l'étape (b), la matière première hydrocarbonée contenant des oléfines est passée sur un ou plusieurs des éléments suivants :
- des tamis moléculaires, de préférence des tamis moléculaires 13X,
- des oxydes métalliques,
- de l'alumine.
